# EUROPEAN PATENT APPLICATION

(11) **EP 1 614 748 A1**
(43) Date of publication of application: **11.01.2006**
(21) Application number: 04076981.2
(22) Date of filing: 08.07.2004
(51) Int. Cl.: C12N 9/24, C12N 15/56, C12N 15/80, C12R 1/00, A01H 5/00

(54) **Fungal polygalacturonase with improved maceration properties**

(71) Applicant: Wageningen University, 6703 HA Wageningen (NL); Stichting voor de Technische Wetenschappen, 3527 JP Utrecht (NL)
(72) Inventor: Benen, Jaques Alex Erwin, 6605 WD Wychen (NL); Joosten, Rob, 6709 PT Wageningen (NL); Kars, Ilona, 6701 AT Wageningen (NL); Krooshof, Geja Hermien, 6708 PM Wageningen (NL); Sibbel-Wagemakers, Cornelia Antonia Maria, 6701 CC Wageningen (NL); Van Kan, Johannes Arnoldus Laurentius, 3911 JP Rhenen (NL)
(74) Representative: van Westenbrugge, Andries

(57) **Abstract**

The present invention relates to an endo-polygalacturonase from the phytopathogenic fungus *Botrytis cinerea* and related enzymes, as well as nucleic acids encoding these enzymes, vectors comprising the nucleic acids and host cells for the production of the enzymes. The invention further relates to methods for producing these polygalacturonases and to methods in which the enzymes are used for hydrolysing internal glycosidic linkages in a polygalacturonic acid chain in a plant material. Such methods are useful in the production of food or feed products, in the extraction of sustances from plant material and in breeding plants having increased resistance towards *Botrytis* fungi.

## Description

### FIELD OF THE INVENTION

The present invention relates to a novel endo-polygalacturonase (BcPG2.2) from *Botrytis cinerea*, as well as to other functional endo-polygalacturonases and their use to degrade pectin or pectin comprising compounds. Provided are also compositions comprising suitable amounts of BcPG2.2 and/or other functional endo-polygalacturonases for industrial processes, such as for methods for preparing food / feed or detergents.

### BACKGROUND OF THE INVENTION

Plant cell walls comprise pectin polymers as an important constituent of the middle lamella. Pectin polymers are mainly composed of chains of 1,4-linked α-D-galacturonic acid monomers, forming a polygalacturonic acid (PGA) backbone and methoxylated derivatives thereof. The food and feed processing industry utilizes purified pectin degrading enzymes (pectinases) for a wide range of industrial processes, such as fruit and vegetable processing (e.g. making juices, wine, puree, animal feed, etc.), which make use of the pectinase activity of these enzymes. A variety of pectinases exist, such as exo-polygalacturonases, endo-polygalacturonases, methylesterases, pectate lyases and pectin lyases. A variety of pectinase encoding genes have been cloned from diverse species, such as fungi, bacteria and plants. Many organisms have been found to contain several genes encoding enzymes with different properties, such as different substrate specificities, pH optima, temperature optima, etc. For example, *Botrytis cinerea* strain SAS56, was found to comprise six genes, encoding six different endo-polygalacturonases (Wubben *et al*., 1999, Applied and Env. Microbiol. Vol. 65: 1596-1602). One of these, Bcpg1, has been shown to be a virulence determinant of *Botrytis* strain B05.10 (Ten Have *et al*., 1998, Mol. Plant Microbe Interact. 11: 1009-1016). The role of the other endo-polygalacturonases remains to be determined, but it may be that a coordinated sequence of endopolygalacturonase expression occurs *in vivo* during tissue infection and colonization. Likewise, it is not known from Wubben et al. (1999) which enzymes may have useful industrial properties and, if present, how such properties could be exploited in industrial processes. *B. cinerea* [anamorph; the teleomorph is referred to as *Botryotinia fuckeliana* (De Bary)] is a broad host range, necrotrophic fungal phytopathogen, which causes "grey mould" on over 200 species of dicotyledonous plants. Graminaceous monocotyledonous plants, on the other hand, are relatively poor hosts. *B. cinerea* has been known to produce a range of pectinolytic enzymes, including up to thirteen different polygalacturonases (Van der Cruyssen *et al*. 1994, Meded. Fac. Landbouwkd. Toegep. Biol. Wet. Univ. Gent 59: 894-905).

Endo-polygalacturonases (E.C. 3.2.1.15) hydrolyse the (internal) glycosidic linkages in the polygalacturonic acid chain, forming oligogalacturonic acid fragments. Although a range of cloned genes encoding endo-polygalacturonase enzymes already exist, there is a need for additional enzymes, with enhanced or different enzymatic properties. Due to the diversity of pectin degrading enzymes, it remains a challenge to identify those enzymes which are useful for industrial purposes. In addition, enzyme compositions usually comprise a mixture of different enzymes, so that the overall enzymatic activity is optimized for a certain industrial process. Alone for this purpose the availability of different enzymes with slightly different properties is desirable, in order to be able to chose and combine the ones which complement each other in an optimal way.

### GENERAL DEFINITIONS

"Polygalacturonase activity" refers to the ability to hydrolyse glycosidic linkages in polygalacturonic acid chains. Endo-polygalacturonase (E.C. 3.2.1.15) activity refers to the ability to hydrolyse internal glycosidic linkages in a polygalacturonic acid chain, as opposed to exo-polygalacturonase (E.C. 3.2.1.67) activity which refers to the ability to release terminal polygalacturonic acid from a polygalacturonic acid chain by hydrolysis of the (terminal) glycosidic linkage. Endo-polygalacturonase activity is determined as described in Example 6 herein, whereby 1 unit of endo-polygalacturonase activity is defined as the amount of enzyme capable of releasing 1 µmole of reducing sugar ends per minute from polygalacturonic acid (Sigma) as the model substrate at 30°C in 50 mM sodium acetate buffer, pH 4.2, with 0.25 % (w/v) substrate concentration.
The terms protein and peptide are used herein interchangeably. The term enzyme refers to a protein which has enzymatic activity.
The term "gene" means a DNA fragment comprising a region (transcribed region), which is transcribed into an RNA molecule (e.g. an mRNA) in a cell, operably linked to suitable regulatory regions (e.g. a promoter). A gene may thus comprise several operably linked fragments, such as a promoter, a 5' leader sequence, a coding region and a 3'nontranslated sequence (3'end) comprising a polyadenylation site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. In one embodiment the 5'-end of the coding sequence preferably encodes a secretion signal, so that the encoded protein or peptide is secreted out of the cell. The coding sequence is preferably in sense-orientation and encodes a desired, biologically active protein or protein fragment.
A "chimeric" (or recombinant) gene refers to any gene, which is not normally found in nature in a species, in particular a gene in which different parts of the nucleic acid region are not associated in nature with each other. For example the promoter is not associated in nature with part or all of the transcribed region or with another regulatory region.
A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules.
The term "expression vector" refers to nucleotide sequences that are capable of affecting expression of a gene in host cells or host organisms compatible with such sequences. These expression vectors typically include at least suitable transcription regulatory sequences and optionally, 3' transcription termination signals. Additional factors necessary or helpful in effecting expression may also be present, such as expression enhancer elements. DNA encoding the polypeptides of the present invention will typically be incorporated into the expression vector. The expression vector will be introduced into a suitable host cell and be able to effect expression of the coding sequence in an *in vitro* cell culture of the host cell. The expression vector will be suitable for replication in a eukaryotic host cell or organism, such as a cultured mammalian, plant, insect, yeast, fungi or other eukaryotic cell line, or in a prokaryotic host, such as a bacterial host.
As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is only active in specific types of tissues or cells.
The term "selectable marker" is a term familiar to one of ordinary skill in the art and is used herein to describe any genetic entity which, when expressed, can be used to select for a cell or cells containing the selectable marker. Selectable marker gene products confer for example antibiotic resistance. Genes conferring resistance to antibiotics such as kanamycin, rifampicin, erythromycin, actinomycin, chloramphenicol, tetracyclines, nisin and lactacin F are generally known in the art. The term "reporter" may be used interchangeably with marker, although it is mainly used to refer to visible markers, such as green fluorescent protein (GFP). Selectable markers may be dominant or recessive or bidirectional.
As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.
"Gene delivery" or "gene transfer" refers to methods for reliable introduction of recombinant or foreign DNA into host cells. The transferred DNA can remain non-integrated or preferably integrates into the genome of the host cell. Gene delivery can take place for example by transduction, using viral vectors, or by transformation of host cells, using known methods, such as electroporation, cell bombardment and the like.

A "recombinant micro-organism" refers to a micro-organism comprising a (man made) nucleic acid construct within its cell(s), in particular one or more chimeric genes. The recombinant micro-organism preferably contains the nucleic acid construct or vector as an episomally replicating molecule, or alternatively and more preferably, integrated into its genome. The latter has the advantage of greater genetic stability of the introduced DNA. It is immaterial by what method the nucleic acid construct is introduced into the micro-organism. Suitable transformation methods for introducing nucleic acid constructs into cells of micro-organisms, such as e.g. electroporation, are available to a skilled person.
A "transgene" is herein defined as a gene that has been newly introduced into a cell, i.e. a gene that does not normally occur in the cell. The transgene may comprise sequences that are native to the cell, sequences that naturally do not occur in the cell, and it may comprise combinations of both. A transgene may contain sequences coding for one or more proteins that may be operably linked to appropriate regulatory sequences for expression of the coding sequences in the cell. The transgene may be integrated into the host cell's genome.
The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's) (self-cloning is defined herein as in European Directive 98/81/EC Annex II). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later.
The term "substantially identitical", "substantial identity" or "essentially similar" or "essential similarity" means that two peptide or two nucleotide sequences, when optimally aligned, such as by the programs GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). It is clear than when RNA sequences are said to be essentially similar or have a certain degree of sequence identity with DNA sequences, thymine (T) in the DNA sequence is considered equal to uracil (U) in the RNA sequence.
Sequence alignments and scores for percentage sequence identity may be determined using computer programs, such as the GCG Wisconsin Package, Version 10.3, available from Accelrys Inc., 9685 Scranton Road, San Diego, CA 92121-3752 USA. Alternatively percent similarity or identity may be determined by searching against databases such as FASTA, BLAST, etc.
"Stringent hybridization conditions" can also be used to identify nucleotide sequences, which are essentially similar to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridizes to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridizations (Northern blots using a probe of e.g. 100nt) are for example those which include at least one wash in 0.2X SSC at 63°C for 20min, or equivalent conditions. Stringent conditions for DNA-DNA hybridization (Southern blots using a probe of e.g. 100nt) are for example those which include at least one wash (usually 2) in 0.2X SSC at a temperature of at least 50°C, usually about 55°C, for 20 min, or equivalent conditions.
The term "comprising" is to be interpreted as specifying the presence of the stated parts, steps or components, but does not exclude the presence of one or more additional parts, steps or components. A nucleic acid sequence comprising region X, may thus comprise additional regions, i.e. region X may be embedded in a larger nucleic acid region. In addition, reference to an element by the indefinite article "a" or "an" does not exclude the possibility that more than one of the element is present, unless the context clearly requires that there be one and only one of the elements. The indefinite article "a" or "an" thus usually means "at least one".

### DETAILED DESCRIPTION OF THE INVENTION

The present invention relates to a novel endo-polygalacturonase (endoPG), cloned from the haploid *Botrytis cinerea* strain B05.10, that may be used in industrial processes and industrial enzyme compositions. The endoPG enzyme is referred herein to as BcPG2.2, in order to distinguish it's name from the prior art enzyme termed BcPG2 (Wubben *et al*. 1999, supra, and GenBank Accession number U68716).

It was surprisingly found that BcPG2.2 has enhanced cell maceration properties, compared to known endoPG enzymes, such as the enzyme from *Aspergillus niger* PGII (AnPGII) described by Benen *et al*. (1999, Eur. J. Biochem. 259: 577-585). These enhanced properties make this new enzyme particularly useful for use in food- and feed- processing methods.

The pre-pro form of the BcPG2.2 enzyme, i.e. the immature full-length amino acid sequence of the BcPG2.2 enzyme as encoded in the *B. cinerea* B05.10 genome, comprises 374 amino acids (SEQ ID NO: 2), and is encoded by the cDNA *Bcpg2.2* (SEQ ID NO: 1). The first 19 amino acids comprise a putative signal peptide (according to SignalP Version 3.0, Nielsen *et al*., 1997, Protein Engineering, 10: 1-6 and Dyrløv Bendtsen *et al*., JMB: to appear 2004). The mature peptide is 344 amino acids long (amino acid 31-374 of SEQ ID NO: 2 and SEQ ID NO: 3), due to a dibasic cleavage site (Arg-Arg) between amino acid 31 and 32 of SEQ ID NO: 2, as is known for many other secreted fungal enzymes.

At the amino acid sequence level, the mature protein (SEQ ID NO: 3) has 97 % sequence identity to the known BcPG2 sequence of Wubben *et al*. (1999, supra) (using Needleman and Wunsch global alignment, GAP opening 8.0 and GAP extension 2.0). This difference is due to 7 amino acid differences: amino acid 53 is isoleucine (Ile) in BcPG2.2 instead of a threonine (Thr), amino acid 186 is Asparagine (Asn) in BcPG2.2 instead of Serine (Ser), amino acids at position 277 to position 279 are Aspartic Acid (Asp) - Threonine (Thr) - Lysine (Lys) in BcPG2.2 instead of Glycine (Gly) - Alanine (Ala) - Threonine (Thr), amino acid 283 is Threonine (Thr) in BcPG2.2 instead of Serine (Ser) and amino acid 368 is Valine (Val) in BcPG2.2 instead of Isoleucine (Ile). However, as mentioned above, no industrially useful properties have been ascribed to the various enzymes disclosed by Wubben et al. (1999, supra).

On the other hand, sequence identity to the *Aspergillus tubigensis* polygalacturonase of US 6,602,696B1, an enzyme with useful activity, is only 57%. In one embodiment of the invention therefore, a method of using an enzyme, which has endopolygalacturonase activity and which has at least 58%, 59%, 60%, 65%, 70%, 75%, 80%, 85%, 90%, 95% , 98%, or 98.5% amino acid sequence identity to SEQ ID NO: 3 (referred herein to as as "substantially related sequences" or "substantially related enzymes" or "substantially related proteins"), for preparing a composition for use in industrial processes, is provided. Preferably the enzyme is a synthetic, recombinant or isolated enzyme. Also provided are compositions comprising one or more of these substantially related enzymes. In addition functional fragments of the substantially related enzymes, as further described below, may be used in the methods and compositions. "Industrial processes" refer in particular to food- or feed- production processes or any production process for non-food/feed products, wherein the process involves in at least one step the hydrolysis of internal glycosidic linkages in polygalacturonic acid chains. This step may for example be the maceration or liquefaction of plant tissue components or plant tissue extracts (e.g. cells, whole tissues or organs), such as processes for the preparation of fruit- or vegetable juices and the like (as described further below). The term "Industrial enzyme compositions" comprises two types of compositions. Firstly, compositions comprising at least one functional protein, substantially related protein or fragment or variant according to the invention and whereby the composition is suitable for use in industrial production processes. These compositions may thus be added to a base composition in an industrial production process, irrespective of whether the protein(s) according to the invention are present in the end product. Secondly, end-product compositions are referred to, such as detergent compositions, whereby at least one functional protein according to the invention is present in the end-product in suitable amounts. Depending on the (industrial) process in which the polygalacturonases of the invention are applied, the compositions of the invention may comprise, in addition to a polygalacturonase of the invention, further enzymes having other and complementary activities. Such further enzymes may e.g. include Pectinases, like pectin lyases, pectate lyases, rhamnogalacturonan lyases, rhamnogalacturonan hydrolases, xylogalacturonases, pectin methylesterases, pectic acetylesterases; Pectin side chain degrading enzymes like arabinases, arabinofuranosidases, galactanases, galactosidases; Hemicellulose degrading enzymes like xylanases, arabinoxylanases, glucanases, xyloglucanases; acetyl xylan esterases and/or Cellulose degrading enzymes like cellulases and cellobiohydrolases.

In another embodiment the invention relates to a method for hydrolysing internal glycosidic linkages in a polygalacturonic acid chain in a plant material, the method comprising the step of treating the plant material with an effective amount of a protein that has 58%, 59% 60%, 65%, 70%, 75%, 80%, 85%, 90%, 95%, 98%, 98.5% or more amino acid sequence identity with SEQ ID NO: 3 and that has endo-polygalacturonase activity. Alternatively, the plant material is treated with an effective amount of a composition comprising the protein. Preferably in the method, an effective amount of the protein or the composition comprising the protein is added to the plant material or is brought into contact with the plant material. In a preferred method, the protein is the sole protein that has endo-polygalacturonase activity in the composition or in the treatment of the plant material.

In another embodiment the invention relates to a method for producing a food or a feed product from a plant material comprising a polygalacturonic acid chain. The method preferably comprises the step of hydrolysing internal glycosidic linkages in the plant material as described above. In a preferred method the food or feed product is selected from the group consisting of a vegetable- or fruit-juice, -oil, -wine, -puree (including puree's for incorporation into babyfood), -sauces, -drinks, -jellying product such as a jam or marmalade and animal feed. Similarly, one embodiment of the invention relates to a method for extracting one or more substances or compounds from a plant material comprising a polygalacturonic acid chain, whereby the method preferably comprises the step of hydrolysing internal glycosidic linkages in the plant material as described above. Substances or compounds to be extracted in this method may include oils, dyes, flavours, fragrances and or drugs.

In another embodiment the invention relates to a method for macerating or liquefying a plant material comprising a polygalacturonic acid chain. The method preferably comprises the step of hydrolysing internal glycosidic linkages in the plant material as described above.

In the above methods of the invention the plant material can include, consist of and/or originate from different plant tissue types, including e.g. roots, stems, leaves, tubers, flowers or fruit. Preferably the plant material is selected from the group consisting of fruit, including e.g. apple, pear, cherry, strawberry, raspberry, other berry types, orange, grapefruit, lemon, other citrus fruit, plum, nectarine, apricot, melon, grape, papaya, mango, passion fruit, kiwifruit, pineapple, custard apple, avocado, banana, walnut, hazelnut, and other nut types; vegetables, including e.g. carrot, cucumber, onion, leek, garlic, eschalot, other bulb vegetable types, tomato, pepper, eggplant, courgette, lettuce, broad bean, other bean types, green peas, spinach, endive, rhubarb, artichoke, cauliflower, Brussels sprouts, and other brassica types; ornamentals, including e.g. rose, tulip, lily, geranium, cyclamen, and other potted plants; herbs or medicinal plants, including e.g. rosemary, basil, ginkgo, neem tree; and non-food plant commodities, including e.g. hemp, tobacco, flax, cotton, and rapeseed.

In another embodiment of the invention an isolated, recombinant or synthetic protein is provided, which has endo-polygalacturonase activity and wherein the protein has at least 58%, 59% 60%, 65%, 70%, 75%, 80%, 85%, 90%, 95% , 98%, 98.1%, 98.2%, 98.3%, 98.5%, 98.6%, 98.8%, or 99.0 % sequence identity to SEQ ID NO: 3 (the mature protein), herein referred to as sequences which are "essentially similar to SEQ ID NO: 3" or "variants of SEQ ID NO: 3". In another embodiment of the invention an isolated, recombinant or synthetic protein is provided, which has endopolygalacturonase activity and wherein the protein has at least 58%, 59%, 60%, 65%, 70%, 75%, 80%, 85%, 90%, 95% , 98%, 98.1%, 98.2%, 98.3%, 98.5%, 98.6%, 98.8%, or 99.0% sequence identity to SEQ ID NO: 2 (the pre-pro form) is provided, herein referred to as sequences which are "essentially similar to SEQ ID NO: 2" or "variants of SEQ ID NO: 2". An isolated protein is herein understood to mean that the protein is isolated from its natural environment, i.e. isolated from the organism to which the enzyme is endogenous and/or isolated from a culture broth of the organism to which the enzyme is endogenous. Preferably the protein or a composition comprising the protein is free of other proteins having endo-polygalacturonase activity.

Further, functional fragments of SEQ ID NO: 3, SEQ ID NO: 2 or of any one of the essentially similar sequences, or of the "substantially related enzymes" (described above) are provided. In one embodiment a functional fragment comprises at least 100, preferably at least 150, more preferably at least 200 consecutive amino acids of SEQ ID NO: 2 or 3, or of any one of the essentially similar or substantially related sequences, and has endo-polygalacturonase activity. Amino acid fragments may be generated by amino acid deletion, restriction digestion or by *de novo* chemical synthesis, or by expression from an expression vector. Their functionality may be tested as described below and in the Examples. Particularly included in the invention are functional fragments of SEQ ID NO: 3, SEQ ID NO: 2 or of any one of the essentially similar sequences, or of the "substantially related enzymes" (described above), which have a small number of amino acids (1-10 or any number there inbetween) removed from the N- or C-terminus or both termini, e.g. due to exoproteolytic activity present during the production of the enzymes.

Essentially similar sequences or substantially related sequences may either be isolated from other species, such as other fungi (e.g. *Botrytis* species, *Aspergillus* species, *Phytophthora* species, *Penicillium* species, *Sclerotinia* species, *Humicola* species *Fusarium* species, etc.), yeasts (*Pichia, Hansenula, Saccharomyces,* etc.), bacteria, plants (e.g. *Solanaceae, Brassicaceae, Gramineae,* etc.), etc. using methods known in the art (for example nucleic acid hybridization methods using stringent conditions as defined above, or PCR methods using specific or degenerate primer pairs which hybridize to parts the nucleic acid sequences provided). Alternatively, essentially similar sequences may be chemically synthesized *de novo* using a peptide synthesizer or cloned and expressed from expression vectors as known in the art. For example, single amino acids may be deleted, replaced or inserted, without affecting the functional properties of the endo-polygalacturonase in a negative way. Likewise, essentially similar sequence may be generated by mutagenesis or gene-shuffling methods (as for example described in US2003215859) or may be identified using in databases using in silico analysis, for example by BLAST or FASTA analysis of any of the sequences provided herein against amino acid or nucleic acid databases. In one embodiment the essentially similar sequence or the substantially related enzyme is obtained from a fungal species, especially from a necrotrophic fungus.

The proteins, protein variants, essentially related enzymes or fragments according to the invention have endo-polygalacturonase activity. The endo-polygalacturonase activity can be tested in an endo-polygalacturonase activity assays (enzyme assay), either qualitatively and/or quantitatively, as described in the Examples, by for example incubating a specific amount of protein or protein fragment with a suitable substrate (such as polygalacturonic acid) and assaying the hydrolysis of the substrate using various methods. In this way the smallest active fragment of a protein can also be easily determined by a skilled person.

In addition, the proteins, variants, essentially related enzymes and functional fragments according to the invention have preferably at least equivalent maceration properties to the maceration properties of the endoPG of SEQ ID NO: 2 and/or SEQ ID NO: 3 when using an assay equivalent to that described in the Examples. The maceration properties can be easily tested incubating a protein according to the invention with pieces of fruit, such as tomato fruit, for a certain period of time (e.g. 20, 30, 40, 50 hours or more) and assessing the tissue break down (compared to control samples) either visually or microscopically as described in the Examples.

Also provided is any nucleic acid sequence encoding any one of the above proteins, protein variants, essentially related enzymes or functional fragments of any one of these. Thus, several nucleic acid sequences are encompassed herein for each amino acid sequence, due to the degeneracy of the genetic code. Also, both cDNA, genomic DNA and RNA sequences are provided. When a DNA sequence is depicted in the sequence listing, while an RNA sequence is referred to, such an RNA sequence has the same base sequence as the DNA sequence, with the difference that thymine (T) is replaced by uracil (U). In particular, in one aspect the invention provides an isolated nucleic acid molecule comprising a nucleotide sequence encoding a polypeptide having endo-polygalacturonase activity, whereby the nucleotide sequence is selected from the group consisting of:
(a) a nucleotide sequence encoding a polypeptide comprising an amino acid sequence that has at least 97.1%, 97.2%, 97.5%, 97.8%, 98.0%, 98.5%, or 99.0% sequence identity with the amino acid sequence of SEQ ID NO. 3;
(b) a nucleotide sequence that has at least 98.1%, 98.2%, 98.3%, 98.5%, 98.6%, 98.8%, or 99.0% sequence identity with the nucleotide sequence of SEQ ID NO. 1;
(c) a nucleotide sequence the complementary strand of which hybridises to a nucleic acid molecule sequence of (a) or (b); and,
(d) a nucleotide sequence that differs from the a nucleotide sequence of (c) due to the degeneracy of the genetic code.
Essentially similar nucleotide sequences or substantially related sequences may either be isolated from other species, such as other fungi (e.g. *Botrytis* species, *Aspergillus* species, *Phytophthora* species, *Penicillium* species, *Sclerotinia* species, *Humicola* species *Fusarium* species, etc.), yeasts (*Pichia, Hansenula, Saccharomyces,* etc.), bacteria, plants (e.g. *Solanaceae, Brassicaceae, Gramineae,* etc.), etc. using methods known in the art (for example nucleic acid hybridization methods using stringent conditions as defined above, or PCR methods using specific or degenerate primer pairs which hybridize to parts the nucleic acid sequences provided). Essentially similar sequences may be produced for example, by deletion, replacement or insertion of one ore more codons without affecting the functional properties of the endopolygalacturonase in a negative way. Likewise, essentially similar sequence may be generated by mutagenesis or gene-shuffling methods (as for example described in US2003215859) or may be identified using in databases using in silico analysis, for example by BLAST or FASTA analysis of any of the sequences provided herein against amino acid or nucleic acid databases. In one embodiment the essentially similar sequence or the substantially related enzyme is obtained from a fungal species, especially from a necrotrophic fungus.

In one embodiment of the invention a vector, in particular an expression vector, comprising a nucleic acid sequence which encodes a protein, protein variant, essentially related enzymes or fragment according to the invention is provided. The vector preferably comprises a transcription regulatory sequence functional in the host cell, operably linked to a nucleic acid sequence encoding a protein, protein variant essentially related enzymes or fragment according to the invention and, optionally, further comprising a 3' nontranslated sequence. In a particular aspect the invention relates to a vector, preferably an expression vector, comprising a nucleotide sequence encoding a polypeptide having endo-polygalacturonase activity, whereby the nucleotide sequence is selected from the group consisting of:
(a) a nucleotide sequence encoding a polypeptide comprising an amino acid sequence that has at least 58%, 60%, 65%, 70%, 75%, 80%, 85%, 90%, 95% , 98%, 98.1 %, 98.2%, 98.3%, 98.5%, 98.6%, 98.8%, or 99.0% sequence identity with the amino acid sequence of SEQ ID NO. 3;
(b) a nucleotide sequence that has at least 35%, 45%, 55%, 65%, 70%, 75%, 80%, 85%, 90%, 95% , 98%, 98.1%, 98.2%, 98.3%, 98.5%, 98.6%, 98.8%, or 99.0% sequence identity with the nucleotide sequence of SEQ ID NO. 1;
(c) a nucleotide sequence the complementary strand of which hybridises to a nucleic acid molecule sequence of (a) or (b); and,
(d) a nucleotide sequence that differs from the nucleotide sequence of (c) due to the degeneracy of the genetic code; and,
whereby the vector further comprises a transcription regulatory element capable of regulating expression in a host cell and wherein the transcription regulatory element is operably linked to the nucleotide sequence.

A further embodiment of the invention relates to a host cell transformed with an expression vector of the invention and that is capable of expressing the nucleic acid encoding the protein, protein variant, essentially related enzymes or fragment according to the invention. In one embodiment the host cell is a microorganism, preferably a fungal host, such as a yeast cell selected from the genera *Pichia* (e.g. *Pichia pastoris*), *Hansenula* (e.g. *Hansenula polymorpha*), *Saccharomyces* (e.g. *S. cerevisiae), Kluyveromyces* (e.g. *K. lactis*), *Yarrowia* (e.g. *Y. lipolytica)* and *Schizosaccharomyces* (e.g. *S. pombe*). In a preferred embodiment the host cell is a methylotrophic yeast, such as *Pichia*. In another embodiment the host cell is a filamentous fungal host selected from the genera *Aspergillus, Trichoderma, Fusarium, Penicillium, Neurospora, Chrysosporium, Sporotrichum, Humicola,* and *Acremonium.*

The transcription regulatory sequence is preferably strongly active in the host cell, either constitutively or following induction. A variety of transcription regulatory sequences, i.e. promoters, capable of directing transcription in the host cells of the invention is available to the skilled person (Goosen et al., 1992, In: Handbook of Applied Mycology" 4: "Fungal Biotechnology", and Romanos et al., 1992, Yeast 8: 423). Preferably the promoter sequence is derived from a highly expressed gene. Examples of preferred highly expressed genes from which promoters are preferably derived include but are not limited to genes encoding glycolytic enzymes such as triose-phosphate isomerases (TPI), glyceraldehyde-phosphate dehydrogenases (GAPDH), phosphoglycerate kinases (PGK), pyruvate kinases (PYK), alcohol dehydrogenases (ADH), as well as genes encoding amylases, glucoamylases, xylanases, cellobiohydrolases, beta-galactosidases, alcohol (methanol) oxidases, elongation factors and ribosomal proteins. Specific examples of suitable highly expressed genes include e.g. the LAC4 gene from *Kluyveromyces* sp., the methanol oxidase genes (AOX and MOX) from *Hansenula* and *Pichia*, respectively, the glucoamylase (glaA) genes from *A. niger* and *A.awamori,* the *A.oryzae* TAKA-amylase gene, the *A.nidulans* gpdA gene and the *T.reesei* cellobiohydrolase genes.
For expression in yeast species, such as *Pichia* or *Hansenula* species, for example the strong (methanol inducible) AOX1 promoter of the alcohol oxidase gene of *Pichia* (see US 4,855,231), the *Pichia pastoris* alcohol oxidase II (AOX2 promoter) (Ohi *et al*., Mol. Gen. Genet 243: 489-499, 1994) or the MOX1 promoter *of Hansenula* are suitable. Alternative promoters are the *Pichia* formaldehyde dehydrogenase promoter (FLD) as described in US 6,730,499 and by Shen *et al*. Gene 216: 93-102, 1998, other yeast promoters, such as the 3-phosphoglycerate kinase promoter (PGK), glyceraldehyde-3-phosphate dehydrogenase (GAFDH or GAP) promoter, galactokinase (GAL1, GAL10) promoter, galactoepimerase promoter, and alcohol dehydrogenase (ADH1, ADRIII) promoter, the *Pichia pastoris* YPT1 promoter (Sears et al, Yeast 14: 783-790, 1998).

*Pichia pastoris* is a preferred host cell/organism, as the fermentation technology for *Pichia pastoris* is highly developed. A *Pichia* expression system is, for example, commercially available as a kit from Invitrogen, which uses the promoter and terminator from the AOX1 gene. Other, analogous expression systems may be used. Various expression vectors are available, such as integrative and autonomously replicating vectors (comprising an autonomous replicating sequence or ARS, as for example described in US 4,837,148).

The vector preferably also comprises a selectable marker gene. The selectable marker may be any gene which confers a selectable phenotype upon the host and allows transformed cells to be identified and selected from untransformed cells. Suitable selectable markers which can be used for selection of the transformed host cells are well known to the skilled person (Goosen et al., 1992, In: Handbook of Applied Mycology" 4: "Fungal Biotechnology", and Romanos et al., 1992, Yeast 8: 423). Preferred markers include but are not limited to e.g. versatile marker genes that can be used for transformation of most filamentous fungi and yeasts such as acetamidase genes or cDNAs (the amdS genes or cDNAs from *A.nidulans, A.oryzae, or A. niger),* or genes providing resistance to antibiotics like G418 or hygromycin or phleomycin. Alternatively, more specific selection markers can be used such as auxotrophic markers which require corresponding mutant host strains: e.g. URA3 (from *S. cerevisiae* or analogous genes from other yeasts), pyrG (from *A. nidulans* or *A. niger)* or argB (from *A.nidulans* or *A. niger).* In a more preferred embodiment, the selection marker is deleted from the transformed host cell after introduction of the expression construct in accordance with the methods described in EP-A-0 635 574, so as to obtain transformed host cells capable of producing the polypeptide which are free of selection marker genes.

The selectable marker system may include an auxotrophic mutant methylotrophic yeast strain and a wild type gene which complements the host's defect. Examples of such systems include the *Saccharomyces cerevisiae* or *Pichia pastoris* HIS4 gene which may be used to complement his4 Pichia strains, or the *S. cerevisiae* or *Pichia pastoris* ARG4 gene which may be used to complement *Pichia pastoris* arg mutants, or the *Pichia pastoris* URA3 and ADE1 genes, which may be used to complement *Pichia pastoris* ura3 resp. ade1 mutants. Other selectable marker genes which function in *Pichia pastoris* include the zeo resistance gene, the G418 resistance gene, blasticidin resistance gene, and the like. Integration of the chimeric gene into the genome can be achieved by insertion or a transplacement into the region of the chromosomal AOX1 locus or integration may be targeted to the HIS4 locus.

For secretion of the peptide outside of the cell, into the culture medium, a nucleic acid sequence encoding a secretion peptide may be operably linked to the coding sequence, so that the secretion peptide forms a fusion protein with the N-terminal of the protein, e.g. the mature endoPG protein. Suitable secretion peptides are for example the signal sequences from the *S. cerevisiae* α-factor, or that of the acid phosphatase of *Pichia pastoris*. Other suitable secretion signals may be derived from genes native (homologous) to the host species or heterologous genes, which encode secreted proteins.

For expression in other host cells, such as bacteria, mammalian cell cultures or plant cells, other regulatory elements as known in the art are suitable. Non-limiting examples of bacterial promoters include for example the [beta]-lactamase (penicillinase) promoter, the lactose promoter, the tryptophan (trp) promoter, the araBAD (arabinose) operon promoter, the lambda-derived P1 promoter and N gene ribosome binding site and the hybrid tac promoter derived from sequences of the trp and lac UV5 promoters. Suitable promoters for mammalian cells include for example viral promoters, such as those from Simian Virus 40 (SV40), Rous sarcoma virus (RSV), adenovirus (ADV), and bovine papilloma virus. Suitable promoters for expression in transgenic plants or plant cell cultures include for example the Cauliflower Mosaic Virus promoters (CaMV) and others.

At the 3'end of the coding sequence a 3'nontranslated nucleic acid sequence (3'end) may be added, which may contain one or more transcription termination sites recognized by the host's transcription machinery. The origin of the 3'end is not very critical and various suitable 3'end sequences may be used. For example, the 3'end sequence may be the 3'end native to the polygalacturonase gene according to the invention or the 3'end of the *Pichia* AOX1 gene, the *Pichia* HIS4 gene or the *Pichia* FLD1 gene. Preferably, for expression in yeast, a 3'end of a yeast gene is used, for example of a gene naturally found in the host cell.

In one embodiment of the invention the codon usage of the nucleic acid sequence may be modified and adapted to the codon usage of the host cell. Such adaptation may enhance expression. Codon usage tables for various prokaryotic and eukaryotic species are published in the major DNA sequence databases (e.g. EMBL at Heidelberg, Germany).

In another embodiment of the invention a microorganism, which comprises a nucleic acid sequence which encodes a protein, protein variant, a substantially related enzyme, or a functional protein fragment according to the invention, under control of a suitable promoter is provided. Especially a methylotropic yeast, preferably *Pichia* (e.g. *Pichia pastoris* or another readily transformable *Pichia* species) or *Hansenula* is provided, which, under suitable growth conditions produces high levels of an endo-PG protein according to the invention. The microorganisms can be made by transforming a host strain with a vector as described above and selecting transformed cells. Preferred *Pichia* pastoris host strains are strains GS115 (NRRL Y-15851), GS190 (NRRL Y-18014), PPF1 (NRRL Y-18017), PPY120H, YGC4, and strains derived therefrom.

The vectors can be introduced into the host cell using known methods. For yeast hosts these include, for example, the spheroplast technique, described by Cregg *et al*. 1985, or the whole-cell lithium chloride yeast transformation system, Ito *et al*. (Agric. Biol. Chem. 48:341), modified for use in *Pichia* as described in EP 312,934. Other published methods useful for transformation of the plasmids or linear vectors include US 4,929,555; Hinnen *et al*. Proc. Nat. Acad. Sci. USA 75:1929 (1978); Ito *et al*. J. Bacteriol. 153:163 (1983); US 4,879,231; Sreekrishna *et al*. Gene 59:115 (1987).

Electroporation and PEG1000 whole cell transformation procedures may also be used, as described by Cregg and Russel, Methods in Molecular Biology: *Pichia* Protocols, Humana Press, Totowa, N.J., pp. 27-39 (1998). For filamentous fungi suitable transformation protocols are described in Goosen et al., 1992, In: Handbook of Applied Mycology" 4: "Fungal Biotechnology", and in EP-A-0 635 574. Transformed host cells can be selected by using appropriate techniques including such as culturing auxotrophic cells after transformation in the absence of the biochemical product required (due to the cell's auxotrophy), selection for and detection of a new phenotype, or culturing in the presence of an antibiotic only allows growth of transformants comprising a resistance gene. Transformants can also be selected and/or verified by integration of the expression cassette into the genome, which can be assessed by, e.g., Southern Blot or PCR analysis.
Included herein are any derivatives of the host cell, such as whole organism derived therefrom, or cells derived therefrom by multiplication or growth. Also included are any derivatives of the host organism. In case of host plants, this includes seeds (F1, F2, F3, etc.), tissues, organs, cells. Derivatives can be identified by the presence of the recombinant DNA in the cells.

The host microorganisms is preferably grown under conditions leading to high expression of the coding sequence according to the invention and the production of high amounts of protein, e.g. at least 0.1, 0.5, 1.0, 5.0 or 10 g of protein per litre of culture supernatant. The culturing conditions depend on the host strain and promoter used. Factors such as pH, temperature, nutrients, oxygen, etc. can be optimized as known in the art. The protein may be isolated from the culture (which may for example be a large scale fermenter) using methods known in the art, such as using ion exchange columns, antibody based methods, and the like. Purification methods obviously also depend on whether the protein is secreted into the culture medium or whether it is present within the host cells.

It is understood that other nucleic acid sequences may be co-expressed in the host cell. For example, sequences which modify the glycosylation pattern of proteins may be co-expressed. Co-expression may be achieved by transforming the host cell at the same time or consecutively with different chimeric genes or by natural transfer of a chimeric gene into the host cell (e.g. conjugation) or crossing with an independently transformed organism.

Either the culture medium (e.g. in concentrated form), or the substantially purified protein or the transformed host cells as such (including derivatives thereof) may be used to make compositions according to the invention.

Various compositions are provided, characterized in that they comprise a suitable amount of at least one (functional) endo-polygalacturonase protein, variant, substantially related enzyme or functional fragment according to the invention. The composition may be solid, semi-solid, liquid, in powder form, freeze-dried, granulated, etc., depending on the use. In one embodiment the composition may further comprise additional compounds which aid plant tissue degradation, such as other enzymes, for example other pectinases, cellulases, hemi-cellulases, and the like. The composition may thus comprise two, three or more enzymes with different or overlapping activities.

The "suitable amount" of the protein may also vary depending on the use, but generally the most suitable amount can be easily determined by a skilled person by simply analysing the degree of hydrolysis of glycosidic linkages in polygalacturonic acid chains achieved when the composition is contacted with compositions comprising polygalacturonic acid chains. The compositions may be used to hydrolyse internal glycosidic linkages in a polygalacturonic acid chains, for example to macerate plant tissue or plant extract-comprising compositions, such as fruit- and/or vegetable cell/tissue comprising compositions. They may therefore be used in industrial processes to prepare a food or feed product (as indicated above) from fruit and/or vegetables (as indicated above). Drinks, purees, sauces and the like made using the compositions according to the invention may have a smoother texture than their conventional counterparts.

A composition according to the invention may thus be added in suitable amounts and at a suitable time point in an industrial process (such as a food- or feed- production process) to a base composition comprising polygalacturonic acid chains, for example comprising tissue, cells or extracts of dicotyledoneous plants (such as apple, pear, cherry, strawberry, raspberry, other berry types, orange, grapefruit, lemon, other citrus fruit, plum, nectarine, apricot, melon, grape, papaya, mango, passion fruit, kiwifruit, pineapple, custard apple, avocado, banana, walnut, hazelnut, and other nut typescarrot, cucumber, onion, leek, garlic, eschalot, other bulb vegetable types, tomato, potato, carrot, pepper, eggplant, courgette, lettuce, broad bean, other bean types, green peas, spinach, endive, rhubarb, artichoke, cauliflower, Brussels sprouts, and other brassica types) and/or monocotyledoneous plants (such as corn, wheat, barley, rye, rice, and oat).

In one embodiment the compositions are used as animal feed or are added to animal feed compositions to aid the feed degradation by the animals. In another embodiment the compositions are detergent compositions, which may for example be used to remove stains (comprising polygalacturonic acid chains) from fabrics.

Apart from a suitable amount of one or more proteins, variants, substantially related enzymes or fragments according to the invention, the compositions may further comprise other components, such as other enzymes, stabilizers, vitamins, anti-oxidants, colorants, oxidizing agents, minerals, amino acids, lipids, water, protein components, sugars, enzyme enhancers, flavorings, etc.

In one embodiment the compositions comprise viable (and/or non-viable) host microorganisms (e.g. lyophilized microorganisms), for example for animal feeds, the host cells may be chosen to be viable in the gastrointestinal tract of animal species which is to ingest the composition.

In yet a further embodiment, methods for producing a protein, protein variant, substantially related enzyme or protein fragment according to the invention are provided. Said method comprises expressing a nucleic acid sequence encoding a functional endo-polygalacturonase protein, protein variant, substantially related enzyme or fragment (all as defined) in a host cell, culturing the host cell under conditions which result in the production of said protein and optionally isolating the protein from the culture medium or from the host cells.

Further provided is a method for using at least one protein, protein variant, substantially related enzyme or fragment or a composition comprising a suitable amount thereof in industrial processes, such as fruit- and/or vegetable juice production, puree production, animal feed production, or detergent production.

It is understood that the endopolygalacturonase(s) may still be present in the end-product of the production process (e.g. the juice) or, preferably, it may be removed from the product. The presence or absence in the final product can be easily tested.

The extremely potent macerating properties of the polygalacturonases of the invention have important implications for the infection process of *Botrytis cinerea*, because the pathogen is able to cause extensive rot and maceration in plants during culturing, as well as in harvested commodities during storage. The important role of this polygalacturonase is confirmed by the observation that *B. cinerea* mutants in which the *Bcpg2* gene is deleted have a reduced virulence on a number of host plants. These mutants are unable to produce the polygalacturonase and hence have a reduced ability to grow through the host cell walls and/or kill host cells. In a further aspect of the invention this observation is exploited for plant breeding purposes. A plant having genotype that produces a higher tolerance or resistance to damage caused by the application of a polygalacturonase of the invention (e.g. either by placing enzyme on the leaf surface; by infiltration of an enzyme solution into the leaf; by expressing an endo-polygalacturonase of the invention by means of a heterologous expression system suitable for plants, such as a transient *Agrobacterium tumefaciens* expression assay; by expressing an endo-polygalacturonase of the invention by means of a recombinant plant virus vector, such as Potato Virus X or Tobacco Rattle Virus) will also be more resistant to *B. cinerea* infection. The polygalacturonases of the invention and composition thereof may thus be used in screening plant genotypes for (partial) resistance to *B. cinerea*. Application of the enzyme in a range of concentrations (e.g. from 0.1-10 Units/ml) and visual, biochemical and/or biophysical inspection of treated tissue over a subsequent period of time (e.g. 20 minutes, 1 to several hours or days) should enable the identification of plant genotypes that are more resistant to the polygalacturonase as compared to a reference plant genotype, which is susceptible to *Botrytis cinerea*. Such plants with a higher resistance to the enzyme are also more resistant to infection by the fungus itself. Thus, in this aspect, the invention relates to a method for determining the susceptibility of a plant's tissue to maceration or liquefaction by a polygalacturonase of the invention, the method comprising the steps of: (a) bringing a tissue of the plant into contact with the polygalacturonase; and, (b) determining the amount of maceration or liquefaction of the plant's tissue, or damage to its cells by visual, bio-physical or biochemical means. The plant's tissue preferably is a leaf, stem or fruit. The enzyme is preferably brought into contact with the plant's tissue by placing a composition comprising the enzyme on the surface of the tissue or by infiltration the composition into the tissue, e.g. a leaf. Alternatively, the enzyme may be brought into contact with the plant's tissue by expression of the enzyme in the plant's tissue as described above. For this purpose preferably a transient expression systems is applied. Such systems are known as agro-infiltration or *A. tumefaciens* transient transformation assay (ATTA) (see e.g. Kapila et al., 1997, Plant Science 122: 101-108; Van der Hoorn et al., 2000, Molecular Plant-Microbe Interactions 13: 273-285; and U.S. 6,369,296). A wide variety of means for determining the amount of maceration, liquefaction or damage to the plant's tissues or cells are known to the skilled person, including e.g. visual inspection by the naked eye or microscopy, (bio)chemical quantification of cell wall or intracellular components released by the action of the polygalacturonase, as well as measurements of changes in the physical properties of the plant's tissue. Thus, in this embodiment the invention further relates to a method for breeding a plant with increased resistance towards a fungus of the genus *Botrytis,* the method comprising the steps of: (a) determining the susceptibility to maceration or liquefaction by a polygalacturonase of tissue of a plurality of plants in a method as defined above; (b) selecting one or more plants whose tissue has reduced susceptibility to the polygalacturonase; and (c) optionally, further breeding of the plants selected in (b). Preferably, the method is a method for breeding a plant with increased resistance towards *Botrytis cinerea*. More generally the invention relates to the use of a polygalacturonase or a composition comprising the enzyme in breeding a plant or plant variety having increased resistance towards a fungus of the genus *Botrytis*, of which preferably *Botrytis cinerea*.

It is also an object of the invention to provide PCR primers, probes and kits which are suitable for detecting and/or quantifying the presence of nucleic acid sequences and/or amino acid sequences according to the invention in samples or compositions. Primers can be designed using known methods based on any of the sequences provided. They may comprise 16, 17, 18, 19, 20, 22, 25, 32 or more consecutive nucleotides of any of the sequences provided or may be degenerate (not matching perfectly with the sequences). Probes may be generated using fragments of the nucleic acid sequences provided. They may be used in nucleic acid hybridization methods known in the art. Kits may comprise primer pairs or probes, additional reagents and protocols and/or antibodies which specifically detect any of the sequences according to the invention.

### FIGURE LEGENDS

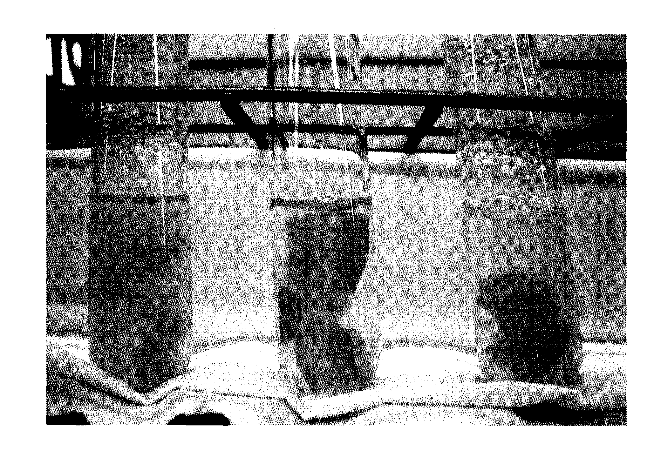

Figure 1: Pieces of tomato fruit incubated for 3 hours with 25 U of BcPG2 (left), buffer (middle), and 25 U of AnPGII (right)

### EXAMPLES

The following non-limiting Examples describe endo-polygalacturonases and methods according to the invention. Unless stated otherwise in the Examples, all recombinant DNA techniques are carried out according to standard protocols as described in Sambrook *et al*. (1989) *Molecular Cloning: A Laboratory Manual*, Second Edition, Cold Spring Harbor Laboratory Press, and Sambrook and Russell (2001) *Molecular Cloning: A Laboratory Manual,* Third Edition, Cold Spring Harbor Laboratory Press, NY; and in Volumes 1 and 2 of Ausubel *et al*. (1994) *Current Protocols in Molecular Biology, Current Protocols,* USA.

### 1. Strains, plasmids and culture media

*Escherichia coli* DH5α was used for standard DNA manipulations. *Pichia pastoris* GS115 (*his4*) and expression vector pPIC3.5, both purchased from Invitrogen (Carlsbad, CA), were used to overproduce endopolygalacturonase 2.2 (BcPG2.2) from *B. cinerea* strain B05.10. Bacterial and yeast cells were cultured using protocols listed in the manual for the *P. pastoris* Expression kit (Invitrogen). All *P. pastoris* cultures were grown at 30°C. To select *P. pastoris* transformants, minimal dextrose plates were used, which contained 1.34% Yeast Nitrogen Base with ammonium sulfate without amino acids (YNB; Difco, Detroit, MI), 2% D-glucose, 0.4 mg/L biotin, and 1.5 % agar. Mut⁺ phenotype was tested on minimal methanol plates, containing 1.34% YNB, 0.5 % methanol, 0.4 mg/L biotin, and 1.5% agar. Buffered methanol-complex medium was used to study recombinant protein production and consisted of minimal methanol medium supplemented with 100 mM potassium phosphate (pH 6.0), 1% yeast extract, and 2% peptone. YPD medium (1% yeast extract, 2% peptone, and 2% dextrose) was used to grow *P. pastoris* GS 115 and prepare frozen stocks of the *P. pastoris* transformants. Fermentation medium contained per liter: 40 g of glycerol, 1 g of CaSO₄·2 H₂O, 14.3 g of K₂SO₄, 11.7 g of MgSO₄·7 H₂O, 4.1 g of KOH, 26.7 mL 85% phosphoric acid, and 4 mL of PMT4 trace elements. The PMT4 trace elements solution contained per liter: 2.0 g CuSO₄·5 H₂O, 0.08 g NaI, 3.0 g MnSO₄·H₂O, 0.2 g Na₂Mo₂O₄·2 H₂O, 0.2 g biotin, 0.02 g H₃BO₄, 0.5 g CaSO₄·2 H₂O, 0.5 g CoCl₂, 7 g ZnCl₂, 22 g FeSO₄·7 H₂O, and 1 mL concentrated H₂SO₄. The fermentor preculture was grown in fermentation medium supplemented with 5 g/L (NH₄)₂SO₄, and 42.9 g/L KH₂PO₄ instead of H₃PO₄.

### 2. Plasmid construction

Standard molecular DNA techniques such as plasmid isolation, restriction analysis and ligations were performed as described by Sambrook *et al*. (1989). DNA fragments were purified from agarose gels with the GFX PCR DNA and Gel Band Purification kit from Amersham Biosciences (Uppsala, Sweden).
Based on known sequences primers to clone the full-length *Bcpg* cDNA from *B. cinerea* B05.10 were designed, see Table 1.

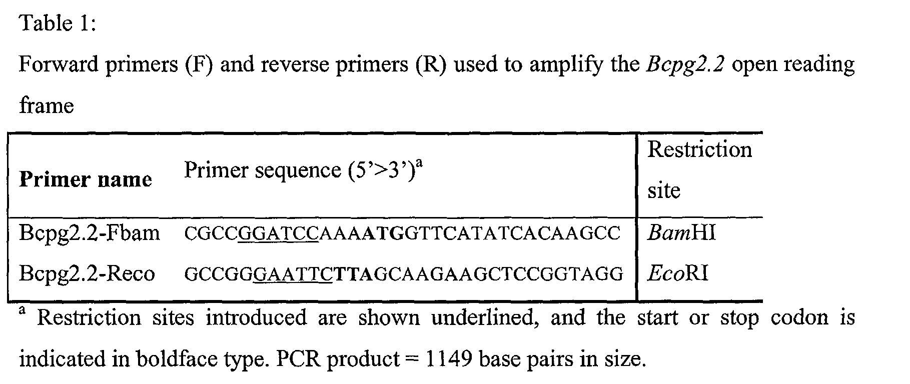

Total RNA isolated from *B. cinerea* B05.10-infected tomato leaves (ten Have *et al*., 2001) was used as a template for RT-PCR. cDNA of *Bcpg2.2* was generated using the primers listed in Table 1 and the Superscript one-step RT-PCR kit (Invitrogen) according to the manufacturer's protocol. The PCR products were cloned behind the strong *AOX1* promoter in the *P. pastoris* expression vector pPIC3.5 using the appropriate restriction enzymes (Table 1). All insert sequences were verified by DNA sequence analysis performed by (Baseclear, Leiden, The Netherlands).

### 3. Expression of recombinant BcPG2.2

pPIC3.5-BcPG2.2 plasmid (5 µg) was linearised using *Sal*I and was used to transform *P. pastoris* GS 115 by electroporation according to the Invitrogen protocol. Electroporation was performed using a Bio-Rad GenePulser set at 1500 V, 25 µF, and 200 Ω, and using 0.2-cm cuvettes. His⁺ transformants were tested for Mut⁺ phenotype and expression of BcPG in liquid buffered methanol-complex medium at 30°C in flasks shaking at 250 rpm. Methanol was added every 24 h to a concentration of 1% (v/v). After four days of growth and induction, expression of BcPG was determined by measuring polygalacturonase activity in the culture medium using a qualitative plate assay. The *P. pastoris* transformant showing the highest expression was used for large-scale BcPG expression and purification.

### 4. Fermentative production of recombinant BcPG in P. pastoris

*P. pastoris* fermentation was performed in a 3-liter jacketed vessel (Applikon, Schiedam, The Netherlands) containing 1.5 L fermentation medium. The temperature was kept at 30°C, agitation at 1200 rpm, and aeration rate at 2 vvm. The pH was controlled at pH 5.0 using a 25% ammonium hydroxide solution, which also served as the nitrogen source. Antifoam 204 (Sigma, A6426) was added manually as required throughout the fermentation. The fermentor medium was inoculated to a start optical density (OD₆₀₀) of 5, using 150 mL of preculture that was grown in shaking flasks at 30°C and 250 rpm for 2 days. The batch culture in the fermentor was grown until the glycerol was completely consumed. Subsequently, a glycerol fed-batch was initiated by feeding 50% glycerol supplemented with 4 mL/L PMT4 trace elements under control of the dissolved oxygen (DO) level. The DO setpoint was 35%, above which glycerol was fed to the culture. When the wet cell weight reached approximately 180 g/L, the glycerol feed was switched to a 100% methanol feed containing 4 mL/L PMT4 trace elements to start the fed-batch induction phase. Similarly, the methanol was fed at dissolved oxygen levels above 35% saturation. The culture was harvested after three days of induction. A wet cell weight of 300 g/L was reached at the end of the fermentation. Cells were discarded and the cell-free supernatant was stored at 4°C or frozen at -20°C until further use. Sodium azide was added to a final concentration of 0.02% (w/v) to prevent microbial growth.

### 5. Purification of recombinant BcPG 2.2

Fermentation culture supernatant was concentrated from 1.5 L to 200 mL using a hollow fiber membrane (H1P10-43; Millipore, Billerica, MA). The concentrated solution was dialysed extensively against 20 mM sodium acetate, pH 4.0. All column materials were obtained from Amersham Bioscience and equilibrated with 20 mM sodium acetate buffer, pH 4.0, before use. The dialysed crude material was first applied to a DEAE-Sepharose FF column (60 mL). BcPG2 activity was found in the flow-through of the DEAE sepharose column as BcPG2 shows a pI of about 9 and, therefore, does not bind to the anion exchange material at pH 4.0. The DEAE flow-through was subsequently loaded onto a Source 30S cation exchange column, and BcPG2 was eluted with a linear gradient of 0-0.35 M NaCl in 20 mM sodium acetate buffer, pH 4.0. Finally, fractions containing purified BcPG2 were pooled, dialysed extensively against 10 mM sodium acetate (pH 4.5), and stored at 4°C after addition of sodium azide (0.02% w/v) to prevent microbial growth. Purified protein was kept at -20°C for prolonged storage.

### 6. Endopolygalacturonase activity assays

Endopolygalacturonase activity was determined qualitatively by spotting a droplet of culture medium on agar plates containing 0.1 % polygalacturonic acid, pH 4 (Sigma, P3850). After incubation at 30°C for at least 30 min, the plate was flooded with 4 M HCl. EndoPG activity was visualized as a white halo in a turbid background. Alternatively, the plates were stained by incubation with a 0.02% ruthenium red solution. In that case, endoPG activity showed as a white spot in a red background.

The catalytic activity of endoPGs was determined quantitatively by means of colorimetric detection of reducing sugar release as described previously (Parenicova et al., 1998). The hydrolysis reactions were carried out at 30°C in 50 mM sodium acetate buffer, pH 4.2, with 0.25 % polygalacturonic acid (Sigma) as the model substrate. Enzyme activity is expressed in U/mg were 1 unit corresponds to 1 µmole of reducing ends liberated per minute.

### 7. Analytical methods

The concentration of purified BcPG2.2 was measured by its absorbance at 280 nm using the absorbance coefficients ε₂₈₀ = 3.34 x 10⁴, as calculated from the amino acid composition. The purity of the isolated enzymes was analysed by SDS-polyacrylamide gel electrophoresis. The molecular mass was estimated using low molecular weight calibration kit 4 (Serva, Heidelberg, Germany). Proteins were visualized by staining with Coomassie brilliant blue R250.

### 8. Improved plant tissue maceration properties

To analyze macerating properties, small pieces of tomato fruit were incubated at room temperature with either 25 U of BcPG2.2, 25 U of *Aspergillus niger* PGII (AnPGII) (Benen *et al*., 1999, Eur. J. Biochem 259, 577-585) in 25 mM NaAc buffer (pH 4.2) or with buffer only. Results are shown in Figure 1. With BcPG2.2, the tomato tissue was almost fully disintegrated within three hours (the tomato skin was still intact). Under the microscope, it appeared as if the cells had dissociated (coherence among the cells was lost), but were still intact. In the case of AnPGII, tissue breakdown was observed but to a significantly lower extent than with BcPG2.2. After 48 hours and after vigorous shaking, the tomato structure was still partly intact with AnPGII. Also, onion was fully disintegrated with BcPG2.2. The BcPG2.2 enzyme also showed good maceration properties when incubated with pieces of carrot or potato.

## Claims

1. A method for hydrolysing internal glycosidic linkages in a polygalacturonic acid chain in a plant material, the method comprising the step of treating the plant material with an effective amount of a composition comprising a protein that has at least 59% amino acid sequence identity with SEQ ID NO: 3 and that has endo-polygalacturonase activity.

2. A method according to claim 1, wherein the protein is the sole protein that has endo-polygalacturonase activity in the composition.

3. A method for producing a food or a feed product from a plant material comprising a polygalacturonic acid chain, the method comprising the step of hydrolysing internal glycosidic linkages according to claim 1.

4. A method according to claim 3, wherein the food or feed product is selected from the group consisting of a juice, an oil, a wine, a puree, a sauce, a drink, a jellying product and animal feed.

5. A method for extracting one or more substances or compounds from a plant material comprising a polygalacturonic acid chain, the method preferably comprises the step of hydrolysing internal glycosidic linkages according to claim 1.

6. A method for macerating or liquefying a plant material comprising a polygalacturonic acid chain, the method comprising the step of hydrolysing internal glycosidic linkages according to claim 1.

7. A method according to any one of claims 1 - 6, wherein the plant material comprises roots, stems, leaves, tubers, flowers or fruit.

8. A method according to any one of claims 1 - 7, wherein the protein is of fungal origin.

9. An isolated protein having endo-polygalacturonase activity and having at least 59% sequence identity to SEQ ID NO: 3.

10. A functional fragment of at least 100 contiguous amino acids of a protein according to claim 9.

11. An isolated nucleic acid molecule comprising a nucleotide sequence encoding a polypeptide having endo-polygalacturonase activity, whereby the nucleotide sequence is selected from the group consisting of:
(a) a nucleotide sequence encoding a polypeptide comprising an amino acid sequence that has at least 97.1 % sequence identity with the amino acid sequence of SEQ ID NO. 3;
(b) a nucleotide sequence that has at least 98.1 % sequence identity with the nucleotide sequence of SEQ ID NO. 1;
(c) a nucleotide sequence the complementary strand of which hybridises to a nucleic acid molecule sequence of (a) or (b); and,
(d) a nucleotide sequence that differs from the a nucleotide sequence of (c) due to the degeneracy of the genetic code.

12. A vector comprising the nucleotide sequence according to claim 11.

13. A vector comprising comprising a nucleotide sequence encoding a polypeptide having endo-polygalacturonase activity, whereby the nucleotide sequence is selected from the group consisting of:
(a) a nucleotide sequence encoding a polypeptide comprising an amino acid sequence that has at least 59 % sequence identity with the amino acid sequence of SEQ ID NO. 3;
(b) a nucleotide sequence that has at least 59% sequence identity with the nucleotide sequence of SEQ ID NO. 1;
(c) a nucleotide sequence the complementary strand of which hybridises to a nucleic acid molecule sequence of (a) or (b); and,
(d) a nucleotide sequence that differs from the a nucleotide sequence of (c) due to the degeneracy of the genetic code; and,
whereby the vector further comprises a transcription regulatory element capable of regulating expression in a host cell and wherein the transcription regulatory element is operably linked to the nucleotide sequence.

14. A host cell comprising a vector as defined in claims 12 or 13.

15. A host cell according to claim 13, wherein the host cell is a microorganism.

16. A method for producing a protein or protein fragment which has endopolygalacturonase activity, said method comprising the steps of:
(a) a host cell as defined in claims 14 or 15 under conditions conducive to the expression of the protein or protein fragment; and,
(b) optionally, recovery of the protein or protein fragment.

17. A method for determining the susceptibility of a plant's tissue to maceration or liquefaction by a endo-polygalacturonase activity having at least 59% sequence identity to SEQ ID NO: 3., the method comprising the steps of:
(a) bringing a tissue of the plant into contact with the polygalacturonase; and,
(b) determining the amount of maceration or liquefaction of the plant's tissue, or damage to its cells by visual, bio-physical or biochemical means.

18. A method for breeding a plant with increased resistance towards a fungus of the genus *Botrytis,* the method comprising the steps of:
(a) determining the susceptibility to maceration or liquefaction by an endopolygalacturonase of tissue of a plurality of plants in a method as defined in claim 17;
(b) selecting one or more plants whose tissues have reduced susceptibility to the polygalacturonase as determined in (a); and,
(c) optionally, further breeding of one or more plants selected in (b).


SE QUENCE LISTING

<110> Stichting voor de Technische Wetenschappen

Wageningen Universiteit & Researchcentrum

<120> Fungal polygalacturonases with improved maceration properties

<130> P215319EP

<140> P215319EP

<141> 2004-07-09

<160> 5

<170> PatentIn version 3.1

<210> 1

<211> 1125

<212> DNA

<213> Botrytis cinerea

<400> 1
atggttcata tcacaagcct tatttctttt ttggccagca ccgctttggt gtctgcagct
60

ccaggttccg ccccagcaga tcttgatagg cgtgctggtt gcacattcag caccgccgct
120

actgctatag cctcaaagac cacctgctct actatcattt tggacagcgt cgttgttcca
180

gctggaacca ctcttgactt gactggcctc aaaaccggca ccaaagttat tttccaggga
240

actgccactt ttggttactc tgagtgggaa ggccctctta tttccatctc cggacaagat
300

attgtcgtca ccggtgcatc cggaa acaag attgacggcg gcggagcaag atggtgggat
360

ggacttggat ctaatgtttc tgccggaaaa ggcaaagtca agcctaagtt cttctcagcc
420

cacaagttga ctggatcttc cagtatcact ggtttgaatt tcctcaatgc acccgttcag
480

tgcatttcta ttggacaatc tgttggattg tcgcttatca atatcaacat tgataa ctct
540

gctggagatg ctggtaacct tggccacaac accgatgctt ttgacatcaa cttgagtcaa
600

aacattttca tctccggagc aattgtcaag aaccaagatg attgtgttgc tgtcaactct
660

ggcaccaaca ttactttcac cggtggaaac tgctctggtg gccatggtct ctccatcggt
720

tccgtcggtg gacgcagcgg caccggagcc aacgatgtca aggacgtgcg attcctctct
780

tctactgttc aaaaatcgac caatggtgtt cgagttaaga ccgtcagcga taccaaaggc
840

tctgtcaccg gcgtcacttt ccaggacatc actctcatcg gcatcaccgg agttggcatt
900

gatgttcaac aggattatca aaatggcagc cctactggta caccaactaa tggtgttcca
960

atcaccggat tgactatgaa caatgttcac ggtaatgtta ttggaggtca aaatacttat
1020

attctctgtg caaactgctc aggctggact tggaataagg ttgctgttac cggtggaact
1080

gttaagaagg cttgtgctgg tgttcctacc ggagcttctt gctaa
1125

<210> 2

<211> 374

<212> PRT

<213> Botrytis cinerea

<400> 2

Met Val His Ile Thr Ser Leu Ile Ser Phe Leu Ala Ser Thr Ala Leu
1               5                   10                  15

Val Ser Ala Ala Pro Gly Ser Ala Pro Ala Asp Leu Asp Arg Arg Ala

            20                  25                  30

Gly Cys Thr Phe Ser Thr Ala Ala Thr Ala Ile Ala Ser Lys Thr Thr
        35                  40                  45

Cys Ser Thr Ile Ile Leu Asp Ser Val Val Val Pro Ala Gly Thr Thr
    50                  55                  60

Leu Asp Leu Thr Gly Leu Lys Thr Gly Thr Lys Val Ile Phe Gln Gly
65                  70                  75                  80

Thr Ala Thr Phe Gly Tyr Ser Glu Trp Glu Gly Pro Leu Ile Ser Ile
                85                  90                  95

Ser Gly Gln Asp Ile Val Val Thr Gly Ala Ser Gly Asn Lys Ile Asp
            100                 105                 110

Gly Gly Gly Ala Arg Trp Trp Asp Gly Leu Gly Ser Asn Val Ser Ala
        115                 120                 125

Gly Lys Gly Lys Val Lys Pro Lys Phe Phe Ser Ala His Lys Leu Thr
    130                 135                 140

Gly Ser Ser Ser Ile Thr Gly Leu Asn Phe Leu Asn Ala Pro Val Gln
145                 150                 155                 160

Cys Ile Ser Ile Gly Gln Ser Val Gly Leu Ser Leu Ile Asn Ile Asn
                165                 170                 175

Ile Asp Asn Ser Ala Gly Asp Ala Gly Asn Leu Gly His Asn Thr Asp
            180                 185                 190

Ala Phe Asp Ile Asn Leu Ser Gln Asn Ile Phe Ile Ser Gly Ala Ile
        195                 200                 205

Val Lys Asn Gln Asp Asp Cys Val Ala Val Asn Ser Gly Thr Asn Ile
    210                 215                 220

Thr Phe Thr Gly Gly Asn Cys Ser Gly Gly His Gly Leu Ser Ile Gly
225                 230                 235                 240

Ser Val Gly Gly Arg Ser Gly Thr Gly Ala Asn Asp Val Lys Asp Val
                245                 250                 255

Arg Phe Leu Ser Ser Thr Val Gln Lys Ser Thr Asn Gly Val Arg Val
            260                 265                 270

Lys Thr Val Ser Asp Thr Lys Gly Ser Val Thr Gly Val Thr Phe Gln
        275                 280                 285

Asp Ile Thr Leu Ile Gly Ile Thr Gly Val Gly Ile Asp Val Gln Gln
    290                 295                 300

Asp Tyr Gln Asn Gly Ser Pro Thr Gly Thr Pro Thr Asn Gly Val Pro
305                 310                 315                 320

Ile Thr Gly Leu Thr Met Asn Asn Val His Gly Asn Val Ile Gly Gly
                325                 330                 335

Gln Asn Thr Tyr Ile Leu Cys Ala Asn Cys Ser Gly Trp Thr Trp Asn
            340                 345                 350

Lys Val Ala Val Thr Gly Gly Thr Val Lys Lys Ala Cys Ala Gly Val
        355                 360                 365

Pro Thr Gly Ala Ser Cys
    370

<210>  3

<211>  343

<212>  PRT

<213>  Botrytis cinerea

<400>  3

Ala Gly Cys Thr Phe Ser Thr Ala Ala Thr Ala Ile Ala Ser Lys Thr
1               5                   10                  15

Thr Cys Ser Thr Ile Ile Leu Asp Ser Val Val Val Pro Ala Gly Thr
            20                  25                  30

Thr Leu Asp Leu Thr Gly Leu Lys Thr Gly Thr Lys Val Ile Phe Gln
        35                  40                  45

Gly Thr Ala Thr Phe Gly Tyr Ser Glu Trp Glu Gly Pro Leu Ile Ser
    50                  55                  60

Ile Ser Gly Gln Asp Ile Val Val Thr Gly Ala Ser Gly Asn Lys Ile
65                  70                  75                  80

Asp Gly Gly Gly Ala Arg Trp Trp Asp Gly Leu Gly Ser Asn Val Ser
                85                  90                  95

Ala Gly Lys Gly Lys Val Lys Pro Lys Phe Phe Ser Ala His Lys Leu
            100                 105                 110

Thr Gly Ser Ser Ser Ile Thr Gly Leu Asn Phe Leu Asn Ala Pro Val
        115                 120                 125

Gln Cys Ile Ser Ile Gly Gln Ser Val Gly Leu Ser Leu Ile Asn Ile
    130                 135                 140

Asn Ile Asp Asn Ser Ala Gly Asp Ala Gly Asn Leu Gly His Asn Thr
145                 150                 155                 160

Asp Ala Phe Asp Ile Asn Leu Ser Gln Asn Ile Phe Ile Ser Gly Ala
                165                 170                 175

Ile Val Lys Asn Gln Asp Asp Cys Val Ala Val Asn Ser Gly Thr Asn
            180                 185                 190

Ile Thr Phe Thr Gly Gly Asn Cys Ser Gly Gly His Gly Leu Ser Ile
        195                 200                 205

Gly Ser Val Gly Gly Arg Ser Gly Thr Gly Ala Asn Asp Val Lys Asp
    210                 215                 220

Val Arg Phe Leu Ser Ser Thr Val Gln Lys Ser Thr Asn Gly Val Arg
225                 230                 235                 240

Val Lys Thr Val Ser Asp Thr Lys Gly Ser Val Thr Gly Val Thr Phe
                245                 250                 255

Gln Asp Ile Thr Leu Ile Gly Ile Thr Gly Val Gly Ile Asp Val Gln
            260                 265                 270

Gln Asp Tyr Gln Asn Gly Ser Pro Thr Gly Thr Pro Thr Asn Gly Val
        275                 280                 285

Pro Ile Thr Gly Leu Thr Met Asn Asn Val His Gly Asn Val Ile Gly
    290                 295                 300

Gly Gln Asn Thr Tyr Ile Leu Cys Ala Asn Cys Ser Gly Trp Thr Trp
305                 310                 315                 320

Asn Lys Val Ala Val Thr Gly Gly Thr Val Lys Lys Ala Cys Ala Gly
                325                 330                 335

Val Pro Thr Gly Ala Ser Cys
            340

<210> 4

<211> 32

<212> DNA

<213> Artificial

<220>

<221> misc_feature

<223> oligonucleotide

<400> 4
cgccggatcc aaaatggttc atatcacaag cc
32

<210> 5

<211> 32

<212> DNA

<213> Artificial

<220>

<221> misc_feature

<223> oligonucleotide

<400> 5
gccgggaatt cttagcaaga agctccggta gg
32